(19) Europäisches Patentamt
European Patent Office
Office européen des brevets

(11) Veröffentlichungsnummer: **0 344 564 A1**

# (12) EUROPÄISCHE PATENTANMELDUNG

(21) Anmeldenummer: **89109153.0**

(22) Anmeldetag: **20.05.89**

(51) Int. Cl.⁴: **A61K 47/00 , A61K 7/00 , A62D 3/00 , C10M 133/00 , A01N 25/32 , C11D 3/00**

(30) Priorität: **31.05.88 DE 3818495**

(43) Veröffentlichungstag der Anmeldung: **06.12.89 Patentblatt 89/49**

(84) Benannte Vertragsstaaten: **ES**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Eisenbrand, Gerhard, Prof. Dr.**
**Eichendorffstrasse 12**
**D-6900 Heidelberg(DE)**
Erfinder: **Sommer, Heidemarie, Dr.**
**Joseph-Haydn-Strasse 16**
**D-6707 Schifferstadt(DE)**
Erfinder: **Wei-Ce, Tang, Prof. Dr.**
**Pfaffenbergstrasse 103**
**D-6750 Kaiserslautern(DE)**

## (54) Verwendung von speziellen Aminen zur Verhinderung der Nitrosaminbildung.

(57) Verwendung von primären Aminen, die strukturbedingt bei Reaktionen mit $NO_x$ nicht zu Nitrosaminen führen oder von sekundären Aminen, die Fängergruppen für die nitrosierenden Agentien enthalten, bei deren Weiterreaktion keine oder nur sehr geringe Mengen Nitrosamine entstehen als Aminbestandteile in Körperpflegemitteln, kosmetischen Zubereitungen zur topikalen Anwendung, technischen Reinigungsmitteln, Kühlschmiermitteln und Hydraulikölen, insbesondere 4-Hydroxycyclohexylamin, p-Aminobenzoesäure, Bis-ß-aminoethylethylendiamin, Trifluorethyl-2-hydroxyethylamin und/oder Bis-(2-hydroxyethyl)aminoethylamin.

EP 0 344 564 A1

## Verwendung von speziellen Aminen zur Verhinderung der Nitrosaminbildung

Die Erfindung betrifft die Verwendung von speziellen Aminen zur Verhinderung der Bildung von krebserzeugenden Nitrosaminen.

Die Entstehung von krebserzeugenden Nitrosaminen insbesondere in kosmetischen Mitteln, Körperpflegemitteln, Kühlschmierflüssigkeiten, äußerlich anzuwendenden Arzneimitteln, technischen Reinigungsmitteln und Hydraulikflüssigkeiten ist ein altes Problem. Exponierte Personen sind Verbraucher von äußerlich anzuwendenden Arzneimitteln, Kosmetik- und Körperpflegemitteln aber auch Beschäftige an bestimmten Arbeitsplätzen, besonders in der metallverarbeitenden Industrie. Für Art und Höhe der Exposition gibt es zahlreiche Literatur. Als Beispiel sei auf eine kürzlich von Sommer und Eisenbrand publizierte Arbeit über kosmetische Mittel hingewiesen (A method for the determination of N-nitrosoalkanolamines in cosmetics, Z. Lebensm. Unters. Forsch. (1988) 186: 234-238, Springer-Verlag 1988).

Bis vor kurzem war in kosmetischen Mitteln die Verwendung von Diethanolamin zugelassen. Seit der Empfehlung des BGA im Frühjahr 1987, Diethanolamin nicht zu verwenden, sind in deutschen Produkten, für welche diese Empfehlung gilt, die Gehalte zurückgegangen. Wie stark letztlich die Verminderung der N-Nitroso-diethanolaminmenge sein wird, muß die Zukunft zeigen, da hier besonders die Lagerzeit eine Rolle spielt. Es ist jedoch bekannt, daß die Nitrosaminproblematik nach wie vor groß ist und daß es schwer sein wird, die ganze Palette der aminhaltigen Produkte auf Gehalte unterhalb der Nachweisgrenze der Nitrosamine (< 10 ppb) zu drücken. Ein besonderes Problem ist es vor allem, Kontakt mit Nitrosierungsmitteln bei Fertigung und Lagerung und letztlich auch nach Auftragen z.B. von Körperlotionen auf die Haut zu meiden. Hier spielen Stickoxide ($NO_x$) die entscheidende Rolle. Weiterhin werden immer noch, nicht nur in Körperpflegemitteln, sondern vor allem Kühlschmiermitteln Konservierungsmittel wie Bronopol (2-Nitro-2-brom-1,3-propandiol) und Bronidox (5-Brom-5-nitro-1,3-dioxan) verwendet, die sehr potente nitrosierende Agentien sind, besonders im alkalischen Bereich (pH > 7).

Als Ergebnis der Situation läßt sich sagen, daß es durch die Vermeidung der Verwendung sekundärer Alkanolamine, die ganz besonders leicht nitrosierbar sind, möglich ist, die Nitrosaminbildung aus den derzeitigen Spitzenbelastungswerten von Hunderten von ppb bis zum ppm-Bereich nach unten zu drücken. Dies gilt bisher aber nur für kosmetische Mittel des deutschen Marktes, bei denen aber natürlich nach wie vor mit der Möglichkeit der Entstehung von Kontaminationen in geringerer Höhe (10-50 ppb) aus dem tertiären Amin Triethanolamin, z.B. bei Kontakt mit $NO_x$, zu rechnen ist. Nach wie vor hohe bis sehr hohe Belastungen mit Nitrosaminen treten bei Kühlschmierstoffen und Hydraulikflüsssigkeiten auf.

Die bei weitem wichtigste Kontamination ist die mit N-Nitrosodiethanolamin, (NDELA) einem potenten Carcinogen, das die Haut überaus gut durchdringt.

Man kann aber die Amine aus den erwähnten Präparaten nicht einfach weglassen, da sie vor allem in kosmetischen Mitteln, aber auch in Kühlschmierflüssigkeiten und Hydraulikflüssigkeiten zur Neutralisation saurer Komponenten, wie Fettsäuren, langkettiger Sulfonsäuren, Phosphorsäurederivaten und in Form von Fettsäureseifen als Emulgator erfoderlich sind.

Es ist auch nicht möglich, statt der bisher verwendeten Trialkanolamine wie z.B. Triethanolamin und tris-2-Hydroxypropylamin

$N(-CH_2-CH_2OH)_3$

$N(-CH_2-\underset{OH}{CH}-CH_3)_3$

einfach durch den verstärkten Einsatz von heute schon verwendeten primären Alkanolaminen zu ersetzen, da hier neben technologischen Nachteilen, wie schlechter Emulgator- und Tensidwirkung, mit $NO_x$ über eine Ammoniumverbindung und als nächste Stufe ein ungestättigtes Hydroxyderivat Acetaldehyd entstehen kann. Der durch Desaminierung gebildete Acetaldehyd reagiert mit überschüssigem Amin und $NO_x$:

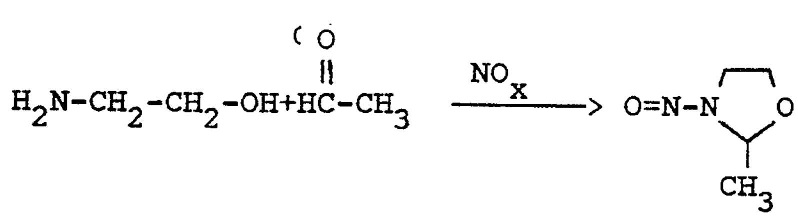


zu einem N-Nitrosooxazolidin-Derivat, das z.B. in Kühlschmiermitteln (R.N. Loeppky et al., Food Chem.

Toxicol. 21 607-613 (1983)) gefunden wurde. Verbindungen dieses Typs sind ebenfalls krebserregend, so daß übliche primäre Amine ebenfalls nicht verwendet werden sollten.

In der EP-0 023 978-A2 wird vorgeschlagen N,N,N′N′-Tetrakis(2-hydroxypropyl)-ethylendiamin zur Unterdrückung der Nitrosaminbildung in kosmetischen Zubereitungen zu verwenden. Dieses eignet sich jedoch nicht im gleichen Maße wie die erfindungsgemäßen primären und sekundären Amine zur Unterdrükkung der Nitrosaminbildung, da bei seiner Reaktion mit $NO_x$ hauptsächlich das Nitrosamin N-Nitroso-N-(2-hydroxypropyl)-N′,N′-bis-(2-hydroxypropyl)-ethylendiamin, dessen biologische Wirksamkeit noch unbekannt ist, neben dem bekanntermaßen carcinogenen N-Nitrosobis(2-hydroxypropyl)amin, gebildet wird.

In der DE-OS-24 11 638 werden Hydroxylgruppen enthaltende, substituierte und unsubstituierte Alkylendiamine oder deren Salze als Haut-Feuchthaltemittel in kosmetischen Zubereitungen beschrieben. Die dort beschriebene allgemeine Formel umfaßt sowohl primäre, sekundäre als auch tertiäre Amine. Der Fachmann entnimmt jedoch dieser Schrift keinen Hinweis darauf, daß einige dieser Amine zur Unterdrükkung der Nitrosaminbildung in kosmeti schen Zubereitungen geeignet sein könnten.

Aufgabe der Erfindung ist die Verwendung solcher Amine, die technologisch gute Ergebnisse erzielen, jedoch beim Abbau keine krebeerregenden Nitrosamine entstehen lassen.

Diese Aufgabe wird gelöst durch die Verwendung solcher Amine, die strukturbedingt bei Reaktionen mit $NO_x$ nicht zur Nitrosaminen führen sondern zu unschädlichen Produkten abgebaut werden oder von Aminen, die Fängergruppen für die nitrosierenden Agentien enthalten, bei deren Weiterreaktion ebenfalls keine Nitrosamine entstehen. Allgemein gesagt, besteht die Erfindung im Einsatz von Aminen, die darauf einwirkende nitrosierende Agentien in unschädliche Verbindungen überführen. Man kann diese Amine einzeln oder im Gemisch verwenden.

Gegenstand der Erfindung ist somit die Verwendung von primären Aminen, die strukturbedingt bei Reaktion mit $NO_x$ nicht zu krebserzeugenden Nitrosaminen führen oder von sekundären Aminen, die Fängergruppen für die nitrosierenden Agentien enthalten, bei deren Weiterreaktion keine oder nur sehr geringe Mengen Nitrosamine entstehen als Aminbestandteile in kosmetischen Zubereitungen, pharmazeutischen Zubereitungen zur topikalen Anwendung, technischen Reinigungsmitteln, Kühlschmiermitteln, Frostschutzmitteln und Hydraulikölen, insbesondere ist Gegenstand der Erfindung die Verwendung von primären Aminen der Formel I

$H_2N$-Q I,

wobei

Q 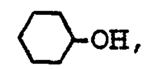, 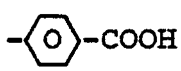 oder einen Rest der Formel

$$-(CH_2)_n-N[CH_2-CHX-R^1]R^3$$

worin

n eine ganze Zahl von 2 bis 6,

X OH oder $NH_2$,

$R^1$ H oder Alkyl mit 1 bis 6 C-Atomen, und

$R^3$ einen unsubstituierten oder mit mindestens einer OH- oder $NH_2$-Gruppe substituierten Alkylrest mit bis zu 18 C-Atomen

darstellen,

bedeutet,

oder von sekundären Aminen der Formel II

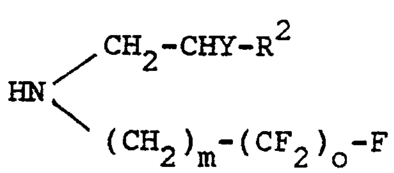 II,

wobei

m 0 oder 1

o 1, 2, 3 oder 4

Y OH oder $NH_2$, und

$R^2$ H oder Alkyl mit 1 bis 6 C-Atomen

bedeuten.

Weiterhin Gegenstand der Erfindung sind kosmetische Zubereitungen mit geringem Nitrosamingehalt, dadurch gekennzeichnet, daß sie als die Nitrosaminbildung unterdrückende Verbindung wenigstens ein primäres Amin der Formel I enthält

$H_2N$-Q I,

wobei Q, n, X, $R^1$ und $R^3$ die genannten Bedeutungen haben, und/oder ein sekundäres Amin der Formel II enthält

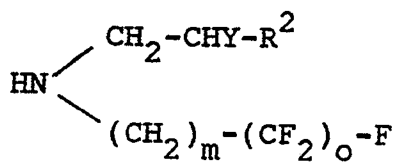

II,

wobei m, o, Y und $R^2$ die genannten Bedeutungen haben.

Gegenstand der Erfindung sind insbesondere solche kosmetische Zubereitung, welche als Konservierungsmittel nitrosierende Agentien enthalten, und solche kosmetischen Zubereitungen, welche das primäre Amin der Formel I in einer Menge von 0,1 bis 0,9 Gewichtsprozent, bezogen auf die gesamte Zubereitung, enthalten.

Gegenstand der Erfindung sind weiterhin Hydrauliköle mit geringem Gehalt an krebserzeugenden Nitrosaminen, welche als die Nitrosaminbildung unterdrückende Verbindung wenigstens ein primäres Amin der Formel I und/oder ein sekundäres Amin der Formel II enthalten und

Kühlschmiermittel mit geringem Gehalt an krebserzeugenden Nitrosaminen, welche als die Nitrosaminbildung unterdrückende Verbindung wenigstens ein primäres Amin der Formel I und/oder ein sekundäres Amin der Formel II enthalten,

technische Reiniger mit geringem Gehalt an krebserzeugenden Nitrosaminen, welche als die Nitrosaminbildung unterdrückende Verbindung wenigstens ein primäres Amin der Formel I und/oder ein sekundäres Amin der Formel II enthalten,

sowie Frostschutzmittel mit geringem Gehalt an krebserzeugenden Nitrosaminen, welche als die Nitrosaminbildung unterdrückende Verbindung wenigstens ein primäres Amin der Formel I und/oder ein sekundäres Amin der Formel II enthalten.

Weiterhin sind pharmazeutische Zusammensetzungen für die topikale Anwendung mit geringem Gehalt an krebserzeugenden Nitrosaminen, welche als die Nitrosaminbildung unter drückende Verbindung wenigstens ein primäres Amin der Formel I und/oder ein sekundäres Amin der Formel II enthält, Gegenstand der Erfindung.

Unter einem geringen Gehalt an krebserzeugenden Nitrosaminen werden voranstehend und im folgenden solche Mengen an Nitrosaminen verstanden, welche unterhalb der Nachweisgrenze der von H. Sommer und G. Eisenbrand (Z. Lebensm. Unters. Forsch. (l988) 186: 234-238) beschriebenen Methode liegen. Die erfindungsgemäßen Mittel enthalten somit 0-20 ppb, vorzugsweise 0-10 ppb, insbesondere 0-5 ppb, an krebserzeugenden Nitrosaminen.

Bevorzugte Alkylendiamine der Formel I sind diejenigen, worin n = 2 ist, und $R^3$ einen Rest der Formel $CH_2$-CHX-$R^1$ darstellt, insbesondere die der Formeln Ia bis If.

$H_2N-CH_2CH_2-N(CH_2-CH_2-OH)_2$ Ia

$H_2N-CH_2CH_2-N(CH_2-CH(OH)-CH_3)_2$ Ib

$H_2N-CH_2CH_2-N(CH_2-CH(OH)-C_2H_5)_2$ Ic

$H_2N-CH_2CH_2-N(CH_2-CH_2-NH_2)_2$ Id

$H_2N-CH_2CH_2-N(CH_2-CH(NH_2)-CH_3)_2$ Ie

$H_2N-CH_2CH_2-N(CH_2-CH(NH_2)-C_2H_5)_2$ If

Weiterhin sind die Verbindungen der Formel I, worin $R^3$ von $CH_2$-CHX-$R^1$ verschieden ist, bevorzugt; insbesondere die Verbindungen der Formel Ig bis Il, worin alkyl vorzugsweise eine längerkettige Alkylgruppe mit bis zu 18 C-Atomen bedeutet:

$H,N-CH_2CH_2-N(CH_2-CH_2-OH)alkyl$ Ig

$H_2N-CH_2CH_2-N(CH_2-CH_2-NH_2)alkyl$ Ih

$H_2N-CH_2CH_2-N(CH_2CH(OH)-CH_3)alkyl$ Ii

$H_2N-cH_2CH_2-N(CH_2CH(NH_2)-CH_3)alkyl$ Ij

$H_2N-CH_2CH_2-N(CH_2CH(OH)-C_2H_5)alkyl$ Ik
$H_2N-CH_2CH_2-N(CH_2CH(NH_2)-C_2H_5)alkyl$ Il

Bei den strukturbedingt unschädlichen Aminen sind besonders zu erwähnen:

1. 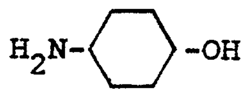 4-Hydroxycyclohexylamin

Hier führt die Reaktion mit $NO_x$ zum Dihydroxyderivat oder Cyclohexen, aber nicht zum Nitrosamin.

2. 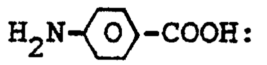: p-Aminobenzoesäure bzw. Anthranilsäure

Die Verbindung kann leicht mit Nitrosierungsmitteln unter Diazoniumionbildung reagieren. Die Weiterreaktion führt jedoch zur p-Hydroxybenzoesäure durch Azokupplung mit anderen Produktbestandteilen, so daß keine Nitrosaminbildung erfolgt. Sie wird als Metallsalz, vor allem als Alkalisalz eingesetzt, wenn die basische Reaktion der Verbindung gewünscht ist,
oder als Salz eines Trialkanolamins oder eines erfindungsgemäßen primären Amins der Formel I oder sekundären Amins der Formel II.

3. $N(-CH_2-CH_2NH_2)_3$ Bis-ß-amino-ethyl-ethylendiamin

Die Verbindung bildet mit $NO_x$ entsprechende Hydroxyethylderivate, also ebenfalls keine Nitrosamine.

4. 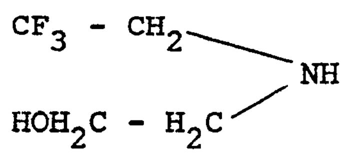

Trifluorethyl-2-hydroxyethylamin

Die Verbindung kann als sekundäres Amin zwar leicht zum Nitrosamin reagieren. Dieses Nitrosamin ist aber, wie Daten zum Metabolismus, zur Mutagenität und Genotoxizität in vitro und in vivo gezeigt haben, nicht gentoxisch (mutagen) und damit mit größter Wahrscheinlichkeit nicht krebserregend.

Als besonderes Beispiel für ein Amin mit Fängergruppe ist zu nennen:

5. $H_2N-CH_2-CH_2-N(-CH_2-CH_2-OH)_2$ N,N-Bis(2-hydroxyethyl)aminoethylamin (BHEAE)

Dies ist ein tertiäres Amin mit zusätzlich primärer Aminogruppe als Fänger für $NO_x$, da die primäre Aminogruppe mit der tertiären um das nitrosierende Reagens konkurriert. Das entstehende Carboniumion reagiert in wässrigem Medium zu Triethanolamin. Die weiterhin mögliche Nitrosaminbildung aus dem Triethanolmin ist dabei jedoch stark erniedrigt, da durch Reaktion mit dem primären Stickstoff schon der Hauptteil des Nitrosierungsmittels abgefangen worden ist.

Die erfindungsgemäß als zur Unterdrückung der Nitrosaminbildung zu verwendenden Verbindungen sind bekannt oder können nach an sich bekannten Verfahren hergestellt werden. Insbesondere können die Alkylendiamine der Formel I, worin Q $-(CH_2)_n-N-[CH_2CHX-R^1]_2$ bedeutet, nach M.S. Malinowskii, Epoxides and their Derivatives, Israel Programs for Scientific Translations, Jerusalem 1965, hergestellt werden.

Kosmetische Zubereitungen, in welchen durch den Zusatz der primären oder sekundären Amine die Nitrosaminbildung erfindungsgemäß unterdrückt wird, sind Lotionen, Salben, Gele, Cremes wie z.B. Tagescremes, Babycremes, Nacht- und Nährcremes, Reinigungscremes, Hautschutzcremes insbesondere Sonnenschutzmittel, Glycerincremes, Cremes mit speziellen Zusätzen tierischer und pflanzlicher Herkunft, Gesichtswasser, Rasierwasser, Bodylotions, Haarpflegemittel, Shampoos, Badezusätze, Dusch- und

Schaumbäder.

Die Einarbeitung in die kosmetischen Zubereitungen kann in an sich bekannter Weise durch einfaches Einrühren bzw. Auflösen erfolgen.

Neben den erfindungsgemäß einzusetzenden primären und sekundären Aminen mit einer die Nitrosaminbildung unterdrückender Wirkung können die kosmetischen Präparationen die in diesen üblicherweise vorhandenen Bestandteilen wie z.B. Wasser, Emulgatoren, natürliche und synthetische Fettsubstanzen, insbesondere Fettsäuren oder Fettsäuregemische, Fettalkohole, Fettsäureester, sowie organische, primäre, sekundäre und tertiäre Amine zur Neutralisation der Carbonsäuren, wie z.B. Monoethanolamin, Diethanolamin, Triethanolamin, Diglykolamin, ionische und nichtionische Tenside, Pflanzenauszüge, Konservierungsmittel, insbesondere Nitrosierungsagentien, Polyether, Duftstoffe und Lösungsmittel in den herkömmlichen Mengen enthalten.

Neben der die Nitrosaminbildung unterdrückenden Wirkung der erfindungsgemäß einzusetzenden Amine, eignen sich diese die in den kosmetischen Zubereitungen enthaltenen Fettsäuren zu neutralisieren und somit den pH-Wert der Zubereitung auf einen der menschlichen Haut angepaßten Bereich einzustellen. Vorzugsweise enthalten diese kosmetischen Zubereitungen nitrosierende Agentien als Konservierungsmittel wie z.B. Bronopol oder Bronidox.

Bevorzugte Fettsäuren in den erfindungsgemäßen kosmetischen Zubereitungen sind z.B. Ölsäure, Linolensäure, Laurinsäure, Stearinsäure, Palmitinsäure oder Isostearinsäure, weiterhin Dicarbonsäuren wie z.B. Azelain- und Sebacinsäure, weiterhin Hydroxycarbonsäuren wie z.B. Milchsäure, Zitronensäure, 12-Hydroxystearinsäure.

Die erfindungsgemäßen Kühlschmiermittel, das sind Bohröle, Schneidöle und Borfette also Kolloide oder echte flüssige Lösungen von Seifen in Mineralölen, welche in der Metallverarbeitenden Industrie bei verschiedenen mechanischen Bearbeitungsverfahren gleichzeitig als Gleitmittel und als Kühlmittel verwendet werden, und die erfindungsgemäßen Reinigungsmittel enthalten neben den zur Unterdrückung der Nitrosaminbildung geeigneten primären und sekundären Aminen Öle natürlicher Herkunft, oder deren Mischung mit Mineralölen, Seifen oder Emulgatoren wie z.B. durch Alkali oder Amine, insbesondere durch die erfindungsgemäßen Amine, neutralisierte synthetische Fettsäuren, Naphthalinsäuren, Naphthalinsulfonsäuren, stearinfreie Fettsäuren, sulfonierte und phenolierte fette Öle und Harze, Alkyl-sulfoxaminsäuren, Alkanolamine wie z.B. Mono-, Di- und Triethanolamin, weiterhin Wasser, Alkohole Polyether, Polyole oder hydrierte Phenole.

Die erfindungsgemäßen Hydrauliköle enthalten neben den die Nitrosaminbildung unterdrückenden primären und sekundären Aminen die dem Fachmann geläufigen Bestandteile wie z.B. Mineralöle oder Mineralöl-Wasser-Emulsionen, Poly-oder Oligoglykolether, Phosphorsäure- oder Kieselsäureester, Silikone oder Fluoralkylether.

Die erfindungsgemäßen Frostschutzmittel enthalten neben dem die Nitrosaminbildung unterdrückenden primären und sekundären Aminen die dem Fachmann geläufigen Bestandteile wie z.B. Wasser, Oligo- oder Polyglykolether, vorzugsweise Ethylenglykol sowie primäre und sekundäre Alkaliphosphate, weiterhin Zusätze von Borax, Sorbit, Alkaliacetat, Glycerin, Benzo-kondensierte Triazole, wie z.B. Benzotriazol oder Tolyltriazol, Alkohole, insbesondere Isopropanol.

Der Anteil der die Nitrosaminbildung unterdrückenden primären und sekundären Amine in den genannten kosmetischen Zubereitungen, Kühlschmiermitteln, Hydraulikölen und Frostschutzmitteln richtet sich nach dem Anteil der zu neutralisierenden Säuren.

Die erfindungsgemäßen pharmazeutischen Zubereitungen für die topikale Anwendung, welche als Arzneimittel in der Human- und Veterinärmedizin verwendet werden, enthalten neben den zur Unterdrükkung der Nitrosaminbildung geeigneten primären und sekundären Aminen und dem geeigneten pharmazeutischen Wirkstoff beispielsweise Wasser, pflanzliche Öle, Kohlenwasserstoffe wie alkylierte Naphthaline, halogenierte Kohlenwasserstoffe wie $CF_2Cl_2$ (z.B. für Aerosole), Benzylalkohole, Polyethylenglykole, Glycerintriacetat, Gelatine, Kohlenhydrate, Magnesiumstearat, Talk, Vaseline, primäre, und tertiäre Amine, wie z.B. Monoethanolamin und Triethanolamin, sowie wasserlösliche synthetische Harze mit gelbildenden Eigenschaften (z.B. solche auf Polyacrylatbasis).

In der Regel enthalten die erfindungsgemäßen Mittel 0.5 bis 25 Gew.-%, vorzugsweise 0.5 bis 15 % insbesondere 1 bis 8 Gew.-% der die Nitrosaminbildung unterdrückenden primären und sekundären Amine.

Weiterhin enthalten die erfindungsgemäßen Mittel vorzugsweise als Konservierungsmittel nitrosierende Agentien.

Daneben sind kosmetische Zubereitungen mit 0,5 bis 0, 9 Gew.-% dieser Amine besonders bevorzugt.

Die nachfolgenden Beispiele sollen den Gegenstand der Erfindung erläutern ohne ihn jedoch hierauf zu beschränken.

Folgende Abkürzungen werden verwendet:

NDELA N-Nitrosodiethanolamin
BHEAE N, N-Bishydroxyethylaminoethylamin
TEA Triethanolamin

Beispiel 1

Um einen Vergleich mit dem heute üblicherweise verwendeten Triethanolamin zu ziehen wird unter praxisnahen Bedingungen, d. h. mit einer, bezogen auf das verwendete min, Zehntel molaren Menge an Nitrit die Nitrosierung durchgeführt. Der Versuchsansatz enthält 20 mM Amin und 12 mM Natriumnitrit. Dabei zeigt BHEAE eine wesentlich geringere NDELA-Bildungsrate als TEA, wie die folgende Tabelle I zeigt:

Tabelle I

| Amin | % NDELA nach 4 h | % NDELA nach 24 h |
|---|---|---|
| BHEAE | $0{,}27 \cdot 10^{-3}$ | $0{,}52 \cdot 10^{-3}$ |
| TEA | $1{,}89 \cdot 10^{-3}$ | $8{,}16 \cdot 10^{-3}$ |

Beispiel 2

Bei der Nitrosierung mit Stickoxiden, wobei l%ige Aminlösungen mit 10 ppm $NO_x$ 4 Stunden begast werden, zeigt BHEAE eine wesentlich geringere NDELA Bildungsrate als TEA, wie die folgende Tabelle II zeigt:

Tabelle II

| Amin | % NDELA nach 4 h |
|---|---|
| BHEAE | $1{,}3 \cdot 10^{-3}$ |
| TEA | $2{,}12 \cdot 10^{-3}$ |

Beispiel 3

Wie schon eingangs erwähnt, werden sowohl in Körperpflegemitteln als auch in Kühlschmiermitteln und hydraulischen Flüssigkeiten, Konservierungsmittel, insbesondere Bronopol, verwendet. Um deren Einfluß zu prüfen wird einer Modellemulsion, bestehend aus: 3,2 % Emulgade F (Cetyl- und Stearylalkohol, Natriumcetyl - und Natriumstearylsulfat), 1,1 % Cetanol, 10,6 % Blauöl, 0,5 % Phenonip (30 % Nipagine in Phenoxyethanol) und 84,6 % Wasser, in einem Fall 2 % BHEAE und im anderen Falle 2 % TEA zugesetzt. Jeder der beiden Ansätze wird geteilt. Einem Ansatz wurde 50 ppm Natriumnitrit und dem anderen Ansatz 0,1 % Bronopol zugegeben.

Nach halbjähriger Lagerung ergaben sich folgende NDELA-Gehalte:

Tabelle III

| Amin | NDELA in Emulsion mit 50 ppm Nitrit | NDELA in Emulsion mit 0,1 % Bronopol |
|---|---|---|
| 2 % TEA<br>2 % BHEAE | 69 ppb<br>< 5 ppb | 875 ppb<br>< 5 ppb |

Die Versuchsergebnisse, insbesondere die vorstehende Tabelle zeigen somit, daß neben den strukturbedingt nur zu unschädlichen Produkten führenden Aminen auch Amine mit Fängergruppe erfolgreich die Nitrosaminbildung anhalten können, selbst wenn das Konservierungsmittel Bronopol vorliegt. Das neue Amin BHEAE erfüllt die Anforderungen, fast kein bzw. nur wenig NDELA unter praxisnahen Bedingungen zu bilden.

Beispiel 4

Um einen Vergleich mit dem in der EP 0 023 978 erwähnten N,N,N′,N′-Tetrakis(2-hydroxypropyl)-ethylene-diamin (THEDA) zu ziehen wird analog Beispiel 1 ein Vergleichsversuch durchgeführt.

Der wässrige Versuchsansatz enthält 20 mM Amin und 20 mM Natriumnitrit und wird 4 Stunden bei 37 °C und pH 4 gehalten. Dabei weist BHEAE eine wesentlich geringere Nitrosaminbildungsrate als THEDA auf, wie die folgende Tabelle IV zeigt:

Tabelle IV

| Amin | % gebildetes Nitrosamin bezogen auf eingesetztes Amin |
|---|---|
| BHEAE<br>THEDA | 0,02<br>1,0 |

**Ansprüche**

1. Verwendung von primären Aminen, die strukturbedingt bei Reaktion mit $NO_x$ nicht zu krebserzeugenden Nitrosaminen führen oder von sekundären Aminen, die Fängergruppen für die nitrosierenden Agentien enthalten, bei deren Weiterreaktion keine oder nur sehr geringe Mengen Nitrosamine entstehen als Aminbestandteile in kosmetischen Zubereitungen, pharmazeutische Zubereitungen für topikale Anwendungen, Kühlschmiermitteln, Frostschutzmitteln und Hydraulikölen.

2. Verwendung von primären Aminen oder sekundären Aminen als Aminbestandteile nach Anspruch 1, dadurch gekennzeichnet,
daß die primären Amine Verbindungen der Formel I sind
$H_2N-Q$    I,
wobei

Q 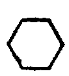-OH, -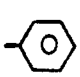-COOH oder einen Rest der Formel

$$-(CH_2)_n-N[CH_2-CHX-R^1]R^3$$

worin
n    eine ganze Zahl von 2 bis 6,
X    OH oder $NH_2$, und

$R^1$ H oder Alkyl mit 1 bis 6 C-Atomen und

$R^3$ einen unsubstituierten oder mit mindestens einer OH oder $NH_2$-Gruppe substituierten Alkylrest mit bis zu 18 C-Atomen

darstellen

bedeutet, und daß die sekundären Amine Verbindungen der Formel II sind

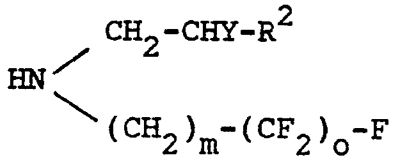

II,

wobei

m 0 oder 1,

o 1, 2, 3 oder 4,

Y OH oder $NH_2$

$R^2$ H oder Alkyl mit 1 bis 6 C-Atomen

bedeutet.

3. Verwendung von 4-Hydroxycyclohexylamin, p-Aminobenzoesäure, Bis-ß-aminoethylethylendiamin, Trifluorethyl-2-hydroxyethylamin und/oder Bis- (2-hydroxyethyl)-aminoethylamin nach Anspruch 1 oder 2.

4. Kosmetische Zubereitung mit geringem Gehalt an krebserzeugenden Nitrosaminen, dadurch gekennzeichnet, daß sie als die Nitrosaminbildung unterdrückende Verbindung wenigstens ein primäres Amin der Formel I

$H_2N$-Q I,

wobei Q, n, X, $R^1$ und $R^3$ die in Anspruch 2 genannten Bedeutungen haben und/oder ein sekundäres Amin der Formel II

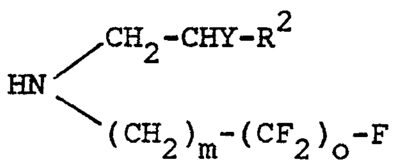

II,

wobei m, o, Y und $R^2$ die in Anspruch 1 genannten Bedeutungen haben,
enthält.

5. Kosmetische Zubereitung nach Anspruch 4, dadurch gekennzeichnet, daß sie als Konservierungsmittel nitrosierende Agentien enthalten.

6. Kosmetische Zubereitung nach Anspruch 4 oder 5, dadurch gekennzeichnet, daß sie das primäre Amin der Formel I in einer Menge von 0,1 bis 0,9 Gewichtsprozent, bezogen auf die gesamte Zubereitung, enthält.

7. Hydrauliköl mit geringem Gehalt an krebserzeugenden Nitrosaminen, dadurch gekennzeichnet, daß es als die Nitrosaminbildung unterdrückende Verbindung wenigstens ein primäres Amin der Formel I nach Anspruch 2 und/oder ein sekundäres Amin der Formel II nach Anspruch 2 enthält.

8. Kühlschmiermittel mit geringem Gehalt an krebserzeugenden Nitrosaminen, dadurch gekennzeichnet, daß es als die Nitrosaminbildung unterdrückende Verbindung wenigstens ein primäres Amin der Formel I nach Anspruch 2 und/oder ein sekundäres Amin der Formel II nach Anspruch 2 enthält.

9. Frostschutzmittel mit geringem Gehalt an krebserzeugenden Nitrosaminen, dadurch gekennzeichnet, daß es als die Nitrosaminbildung unterdrückende Verbindung wenigstens ein primäres Amin der Formel I nach Anspruch 2 und/oder ein sekundäres Amin der Formel II nach Anspruch 2 enthält.

10. Pharmazeutische Zubereitung zur topikalen Anwendung mit geringem Gehalt an krebserzeugenden Nitrosaminen, dadurch gekennzeichnet, daß sie als die Nitrosaminbildung unterdrückende Verbindung wenigstens ein primäres Amin der Formel I nach Anspruch 2 und/oder ein sekundäres Amin der Formel II nach Anspruch 2 enthält.

11. Technisches Reinigungsmittel mit geringem Gehalt an krebserzeugenden Nitrosaminen, dadurch gekennzeichnet, daß es als die Nitrosaminbildung unterdrückende Verbindung wenigstens eine primäres Amin der Formel I nach Anspruch 2 und/oder ein sekundäres Amin der Formel II nach Anspruch 2 enthält.

12. Mittel nach einem der Ansprüche 7 bis 11, dadurch gekennzeichnet, daß es als Konservierungsmittel nitrosierende Agentien enthält.

Europäisches Patentamt

# EUROPÄISCHER RECHERCHENBERICHT

Nummer der Anmeldung

EP 89 10 9153

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X<br><br>A | WO-A-8 302 622 (PCUK PRODUITS CHIMIQUES UGINE KUHLMANN)<br>* Ansprüche * | 1-3,8,12<br><br>4-7,9-11 | A 61 K 47/00<br>A 61 K 7/00<br>A 62 D 3/00<br>C 10 M 133/00<br>A 01 N 25/32<br>C 11 D 3/00 |
| X<br><br>A | FR-A-2 269 327 (HENKEL & CIE GMBH)<br>* Ansprüche * | 1-6,12<br><br>7-11 | |
| X<br><br>A | DE-A-2 331 456 (NIELSEN et al.)<br>* Ansprüche * | 1-6,12<br><br>7-11 | |
| X<br><br>A | EP-A-0 008 171 (UNILEVER N.V.)<br>* Ansprüche * | 1-6,12<br><br>7-11 | |
| A | EP-A-0 243 308 (DISPERSA AG)<br>* insgesamt * | 1-12 | **RECHERCHIERTE SACHGEBIETE (Int. Cl.4)** |
| A | US-A-4 331 468 (M. L. WILLIAMS)<br>* Ansprüche * | 1-12 | A 61 K<br>A 62 D<br>A 01 N<br>C 09 F<br>C 10 M<br>C 11 D |
| A,D | EP-A-0 023 978 (BASF WYANDOTTE CORPORATION)<br>* Ansprüche * | 1-12 | |

Der vorliegende Recherchenbericht wurde für alle Patentansprüche erstellt

| Recherchenort | Abschlußdatum der Recherche | Prüfer |
|---|---|---|
| BERLIN | 04-08-1989 | SIATOU E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)